# EUROPÄISCHE PATENTSCHRIFT

(11) **EP 1 355 898 B1**
(45) Veröffentlichungstag und Bekanntmachung des Hinweises auf die Patenterteilung: **12.10.2005**
(21) Anmeldenummer: 02708275.9
(22) Anmeldetag: 12.01.2002
(51) Int. Cl.: C07D 311/30, C07D 493/04

(54) **VERFAHREN ZUR HERSTELLUNG VON FLAVON DERIVATEN**
METHOD FOR PRODUCING FLAVONE DERIVATIVES
PROCEDE DE FABRICATION DE DERIVES DE LA FLAVONE

(30) Priorität: 01.02.2001 DE 10104350
(43) Veröffentlichungstag der Anmeldung: 29.10.2003
(73) Patentinhaber: Merck Patent GmbH, 64293 Darmstadt (DE)
(72) Erfinder: BUCHHOLZ, Herwig, 60599 Frankfurt (DE); PERRUCHON, Sophie, 67310 Wasselonne (FR)
(86) Internationale Anmeldenummer: PCT/EP2002/000233
(87) Internationale Veröffentlichungsnummer: WO 2002/060889

(56) Entgegenhaltungen:
- EP-A- 0 832 886
- D. NAGARATHNAM: "A SHORT AND FACILE SYNTHETIC ROUTE TO HYDROXYLATED FLAVONES." JOURNAL OF ORGANIC CHEMISTRY., Bd. 56, 1991, Seiten 4884-7, XP002202138 AMERICAN CHEMICAL SOCIETY. EASTON., US ISSN: 0022-3263 in der Anmeldung erwähnt

## Beschreibung

Die vorliegende Erfindung betrifft ein Verfahren zur Herstellung von Flavon Derivaten, bei dem eine 2-Hydroxyacetophenon-Verbindung mit einer Lithiumverbindung metalliert und anschließend mit einer Ketoverbindung umgesetzt wird.

Polyphenolverbindungen, beispielsweise Ring-A hydroxylierte Flavone sind Gegenstand intensiver Forschung und Entwicklungsarbeiten aufgrund ihrer vielfältigen Eigenschaften. Derartige Eigenschaften sind unter anderem die Fähigkeit, retrovirale umgekehrte Transkriptasen, einschließlich HIV-Transkriptase zu blockieren, ebenso wie die Eigenschaft Protein-Tyrosinkineasen und Serin/Threonin-Kineasen zu blockieren. Darüberhinaus weisen derartige Verbindungen Antikrebs- und chemopräventive Eigenschaften auf.

Einige Ring-A hydroxylierte Flavone blockieren ebenso die durch HIV hervorgerufene Syncytiumbildung. Weiterhin haben diese Flavone antioxidative Eigenschaften und finden sich als Zusatzstoffe in Nahrungsmitteln und Kosmetika.

Zur Herstellung von Flavonen und deren Derivaten sind einige Verfahren bekannt, die allesamt mehrere, teilweise aufwendige Syntheseschritte umfassen und beispielsweise in der Veröffentlichung von M. Cushman und D. Nagarathnam in: Tetrahedron Letters, 31, 6497-6500, 1990 beschrieben sind. Diese erweisen sich insbesondere als nachteilig bei der Synthese von Ring-A hydroxylierten Flavonen, weil die phenolischen Hydroxylgruppen der Zwischenverbindungen als Ester oder Ether geschützt werden müssen, um unerwünschten Substitutionsmustern vorzubeugen. Diese Schutzgruppen müssen später wieder in einem weiteren Reaktionsschritt abgespalten werden. Weiterhin tritt oftmals eine teilweise Abspaltung der Schutzgruppen während der weiteren aufeinanderfolgenden Syntheseschritte auf, was zum einen die Gesamtausbeute verringert und die Isolierung des gewünschten Endproduktes aus einem Produktgemisch erschwert.

Um diese Nachteile bei der Synthese von Flavonen zu vermeiden, haben M.Cushman und D. Nagarathnam in der vorerwähnten Veröffentlichung sowie in "Journal of Organic Chemistry", 56, 4884-4887, 1991 vorgeschlagen, mit einem großen Überschuß an Lithiumbis(trimethylsilyl)amid unter homogenen Reaktionsbedingungen die phenolischen Hydroxylgruppen zu deprotonieren und das Lithiumenolat des entsprechenden Ketons herzustellen. Anschließend wird das Kohlenstoffatom des Lithiumenolates regioselektiv mit einem Aroylchlorid acyliert, um so direkt ein β-Diketon Zwischenprodukt zu erhalten, welches anschließend im sauren Medium cyclisiert wird. Nachteilig an diesem Verfahren ist der große Überschuß an der Lithiumbase, der auch bei mehreren Reinigungsschritten nur schwer entfernbar ist, sowie der hohe Preis der Lithiumbase.

Es bestand daher die Aufgabe, ein Verfahren zur Herstellung von Flavonderivaten zu schaffen, welches die vorerwähnten Nachteile des Standes der Technik vermeidet und insbesondere einen einfachen und kostengünstigen Zugang zu Flavonderivaten ohne aufwendige Reinigungsschritte ermöglicht.

Diese Aufgabe wird bei dem erfindungsmäßen Verfahren dadurch gelöst, daß das Verhältnis der molaren Äquivalente von Lithiumverbindung zu den zu metallierenden funktionellen Gruppen der 2-Hydroxyacetophenon-Verbindung 1 bis 1,2 beträgt.

Überraschenderweise wurde gefunden, dass vorerwähntes Verhältnis eine vollständige Metallierung aller Hydroxylgruppen und der Carbonylgruppe der 2-Hydroxyacetophenon Verbindung erlaubt. Weniger als ein Verhältnis von 1 würde zu einer unvollständigen Metallierung führen und damit zu einer großen Anzahl an unerwünschten Nebenprodukten. Ein Verhältnis von mehr als 1,2 hingegen bedeutet den Einsatz einer größeren Menge der zumeist nicht preiswerten Lithiumverbindungen und das Mitschleppen von Lithiumverbindungen bei allen weiteren Folge- insbesondere Reinigungsschritten.

Zudem hat sich herausgestellt dass sich die Reaktion vorteilhaft mit anorganischen Lithiumverbindungen durch führen lässt, da diese preiswert und einfach in großen Mengen verfügbar sind. Des weiteren bieten sie den Vorteil, dass sie in organischen Lösungsmitteln wenig bis gar nicht löslich sind, so dass sie nach einer unter heterogenen Bedingungen geführten Metallisierungsreaktion leicht aus der Reaktionsmischung filtriert werden können, wenn sie im Überschuß eingesetzt werden.

In einer bevorzugten Ausführungsform des erfindungsgemäßen Verfahrens beträgt das Verhältnis von Lithiumverbindung zu den zu metallierenden funktionellen Gruppen der 2-Hydroxyacetophenon-Verbindung genau 1 beträgt. Damit wird erreicht, dass keine evtl. noch gelösten Lithiumverbindungen als Verunreinigungen im Endprodukt auftreten, da diese meist auch nicht durch Reinigungsschritte, wie Umkristallisieren aus den Zwischen- und Endprodukten entfernt werden können.

Vorteilhafterweise wird die Metallierung in einem etherischen Lösungsmittel durchgeführt, da dies die Metallierungsreaktion durch seine Polarität durch Ausbildung von Li-solvaten unterstützt, wodurch die Basizität der Lithiumbase gesteigert wird.

Das im Rahmen des erfindungsgemäßen Verfahrens eingesetzte 2-Hydroxyacetophenon weist bevorzugt folgende Struktur auf: 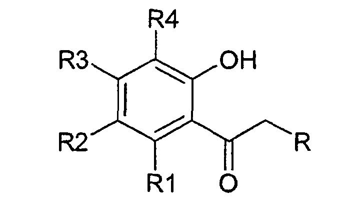 wobei R₁ bis R₄ Wasserstoff, eine funktionelle Gruppe, die aus Hydroxyl, Alkyl, Alkenyl, Ether, Ester, Aryl, O-glycosyl, Alkyloxy, Alkenoxy, Aryloxy, Halogen, Nitro oder Amino ausgewählt ist, bedeuten und gleich oder verschieden sein können und/oder für Rₙ-Rₙ₊₁ für n = 1, 2, 3, Teil eines Ringsystems sein können, und wobei R Wasserstoff oder eine Alkoxy Gruppe bedeutet.

Falls ein Isoflavonderivat hergestellt werden soll, kann R auch eine Phenyl- oder substituierte Phenylgruppe, wie z.B. 4.Methoxyphenyl bedeuten.

Die Ketoverbindung zur Durchführung des erfindungsgemäßen Verfahrens weist folgende Struktur auf: 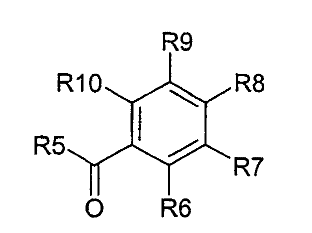 wobei R₆ bis R₁₀ Wasserstoff, eine funktionelle Gruppe, die aus Hydroxyl, Alkyl, Alkenyl, Ether, Ester, Aryl, O-glycosyl, Alkyloxy, Alkenoxy, Aryloxy, Halogen, Nitro oder Amino ausgewählt ist, bedeuten und gleich oder verschieden sein können und/oder wobei Rₙ-Rₙ₊₁ für n = 6, 7, 8, 9 Teil eines Ringsystems sein können, und wobei R₅ eine Halogenid-, Alkoxyl- oder Ester-Gruppe bedeuten kann.

Somit erlaubt die große Variabilität der eingesetzten Ausgangsprodukte, dass nahezu alle bekannte Flavone, Flavonole und von ihnen abstammende Flavonoide mittels des erfindungsgemäßen Verfahren einfach und kostengünstig in einer Eintopfreaktion hergestellt werden können.

Vorzugsweise sind die Hydroxylgruppen der 2-Hydroxyacetophenon Verbindung ungeschützt. Damit werden aufwendige Reaktionen zum Aufbringen und Entfernen von Schutzgruppen vermieden, so dass die Reaktion besonders einfach verläuft.

Im Rahmen der vorliegenden Erfindung werden als Flavonderivate zunächst solche Verbindungen bezeichnet, denen das Grundgerüst des Flavons (2-Phenyl-4H-1-benzopyran-4-on) gemeinsam ist. Darunter fallen im vorliegenden Rahmen auch weiterhin Flavonole mit dem Grundgerüst von 2-Phenyl-3-Hydroxy-4H-1-benzopyran-4-on und Flavonoide, das heißt z.B. Glykoside von Flavonen bzw. Flavonolen, die in Pflanzen weit verbreitete Farbstoffe sind und bei denen ein Kohlenhydratrest, wie z.B. Glucose an die Hydroxylgruppe in Position 3 des Flavonols gebunden ist. Flavone und Flavonderivate weisen sowohl am Ring A wie auch am Phenylring in 2-Position zumeist eine oder mehrere Hydroxylgruppen auf. Weiterhin fallen unter die erfindungsgemäß verwendete Definition auch Isoflavone (3-Phenyl-4H-1-benzopyran-4-on), Chromone und Aurone.

Beispiele für die mittels des erfindungsgemäßen Verfahrens hergestellten Flavonderivate umfassen neben den in den nachstehenden Ausführungsbeispielen erwähnten desweiteren als nicht vollständige und nicht beschränkende Aufzählung Apigenin, Acacetin, Chrysin, Flavon, 7, 4-Dihydroxyflavon, 7, 3',4'-Trihydroxyflavon, 6-Hydroxy-4'-methoxyflavon, Luteolin, Diosmetin, Tricetin, Hypolaetin, Prosogerin, 3, 7-Dihydroxyflavon, Luteolin-3', 4',5'-trimethylether, 7, 3', 4', 5'-Tetrahydroxyflavon, Tricetin-3',4',5'-trimethylether,Resokaempferol, Kaempferol, Isokaemferid, Kaempferid, Ermanin, Fisetin, Herbacetin, 3,7,8,4'-Tetrahydroxyflavon, Quercetin, Dillenetin, Transilitin, Gossypetin, Myricetin, Annulatin, Hibiscetin.

Flavone, insbesondere Flavonoide bzw. Flavonoidgemische werden beispielsweise in der Lebensmittel- und Kosmetikindustrie verwendet und gewinnen dort zunehmend an Bedeutung. Besonders monoglykosidierte Flavonoide wie z.B. Isoquercetin zeichnen sich dabei durch eine gute Aufnahmefähigkeit im menschlichen Körper aus.

Weitere Vorteile und Ausgestaltungen der Erfindung ergeben sich aus der Beschreibung und den Ausführungsbeispielen.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Allgemein gesprochen handelt es sich bei dem erfindungsgemäßen Verfahren um eine Eintopfsynthese von Flavonderivaten gemäß nachstehender Reaktion: 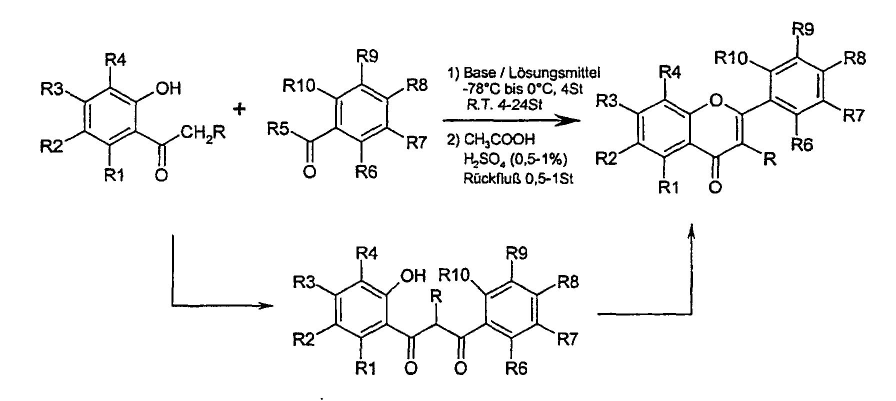 wobei R im Falle der 2-Hydroxyacetophenonverbindung Wasserstoff oder eine Alkoxylgruppe sein kann. Wenn R Wasserstoff ist entsteht ein Flavon im engeren Sinn, im Falle einer Alkoxylgruppe entsteht ein Flavonol.

Im Falle der Ketoverbindungen bedeutet R₅ Chlorid, d.h. die Verbindung ist ein Säurechlorid, eine Alkoxylgruppe, d.h. die Verbindung ist ein Ester, oder eine Estergruppe, d.h. die Verbindung ist ein Säureanhydrid. Die Verwendung unterschiedlicher Gruppen erlaubt je nach eingesetztem Substrat auch die Variation und genaue Wahl der Reaktionszeit. Beispielsweise beträgt die Reaktionszeit bei Verwendung eines Säurechlorids oder eines Säureanhydrids zwischen 2-6 Stunden, meistens zwischen 4-5 Stunden. Bei Verwendnung eines Esters oder unter Verwendnung silylierter Schutzgruppen beträgt die Reaktionszeit mehr als 8 Stunden, zumeist mehr als 10, oftmals jedoch auch etwa 16-20 Stunden.

Zunächst wird die 2-Hydroxyacetophenon Verbindung mit dem Keton und einer Lithiumverbindung in trockenem THF bei niedrigen Temperaturen kondensiert (-78°C bis -50 °C) und ergibt ein stabiles Diketon Zwischenprodukt. Bei Temperaturen von mehr als -50°C verläuft die Reaktion entweder gar nicht mehr oder zu schnell, d.h. mit unerwünschten Nebenprodukten oder aber unter Zersetzung des Ausgangsproduktes ab, so daß der Bereich von -78°C bis -50 °C bevorzugt ist. Anschließend wird das Diketon unter sauren Bedingungen bei 95-100°C cyclisiert, um ein Flavonderivat zu ergeben.

Dabei können **R**_{**1**}**, R**_{**2**}**, R**_{**3**}**, R**_{**4**}**, R, R**_{**6**}**, R**_{**7**}**, R**_{**8**}**, R**_{**9**}**,** and **R**_{**10**} Wasserstoff, eine funktionelle Gruppe, die aus Hydroxyl, Alkyl, Alkenyl, Ether, Ester, Aryl, O-glycosyl, Alkyloxy, Alkenoxy, Aryloxy, Halogen, Nitro oder Amino ausgewählt ist, bedeuten und gleich oder verschieden sein können und/oder wobei Rₙ-Rₙ₊₁ für n = 1, 2, 3 und/oder 6, 7, 8, 9 Teil eines Ringsystems sein können.

Als Lithiumbasen, die in einem erfindungsgemäßen Verfahren Verwendung finden, sind die nachstehend aufgeführten Lithiumbasen besonders geeignet:
LiClO, LiClO₄, LilO₄, Li₂O, LiOH, Li₂CO₃.

Als Lösungsmittel für die Durchführung der Metallisierungsreaktion wird wie vorstehend beschrieben bevorzugt ein etherisches Lösungsmittel, beispielsweise Diethylether, Tetrahydrofuran (THF), Dibutylether. Andere polare Lösungsmittel, wie Methylethylketon und dergleichen können jedoch ebenfalls verwendet werden, aber auch je nach eingesetztem Hydroxyacetophenon apolare Lösungsmittel, wie z.B. n-Hexan, Heptan, Benzol, Toluol usw.

Zur weiteren Verdeutlichung der Erfindung wird auf die nachstehenden Ausführungsbeispiele verwiesen:

Die Bezugsquellen für die verwendeten Substanzen sind wie folgt:

| **Name** | **Hersteller** | **Artikelnr.** | **Reinheit** |
|---|---|---|---|
| Lithiumhydroxid | Merck KGaA | 105691 | LAB 98% |
| THF | Merck KGaA | 108107 | Seccolov getrocknet |
| Methanol | Merck KGaA | 106009 | Pro analysi |
| Chloroform | Merck KGaA | 102445 | Pro analysi |
| Salzsäure rauchend | Merck KGaA | 100314 | Reinst 37% |
| Schwefelsäure | Merck KGaA | 100731 | Pro analysi 95-97% |
| Eisessig | Merck KGaA | 100063 | Pro analysi 100% |
| 4-Methoxy benzoesäure chlorid | Merck KGaA | 820106 | Zur Synthese >98% |
| 3,4-Dimethoxy benzoesäurechlorid | Aldrich | 25,804-0 | 98% |
| Isophthalatchlorid | Merck KGaA | 804808 | Zur Synthese>99% |
| 9-Fluorenon-4-carbonsäurechiorid | Aldrich | 24,958-0 | 97% |
| 2,4-Dihydroxyacetophenon | Merck KGaA | 822027 | Zur Synthese>98% |
| 2,5- Dihydroxyacetophenon | Merck KGaA | 818284 | Zur Synthese >98% |
| 2,6- Dihydroxyacetophenon | Merck KGaA | 820472 | Zur Synthese >99% |
| 2,4,6-Trihydroxyacetophenon | Aldrich | T-6,480-2 | Hydrat, 98% |
| Piperonylbenzoesäurechlorid | Aldrich | 37,889-5 | 99% |
| 4-Chlorobenzoesäurechlorid | Merck KGaA | 802618 | >98% |
| 4-Nitrobenzoesäurechlorid | Merck KGaA | 806772 | >98% |

### Beispiel 1

Trockenes, pulverförmiges Lithiumhydroxid (29,1 mmol, 5 Äquivalente) wird auf einmal zu einer gut gerührten Lösung von 2,4,6-Trihydroxyacetophenon (5,828 mmol) in trockenem THF (5 ml) unter Argonatmosphäre bei -78°C zugegeben. Die Reaktionsmischung wird bei -78°C während einer Stunde gerührt und anschließend während zwei Stunden bei ca. 0°C. Nach erneutem Abkühlen auf -78°C, wird auf einmal eine Lösung von 3,4-Dimethoxybenzoesäurechlorid (5,9 mmol) in THF (10 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (150g) und konzentrierter HCl (10 ml) geleert und mit Chloroform (3x50 ml) extrahiert. Die Lösungsmittel werden aus den getrockneten Extrakten entfernt und der Rückstand unter Vakuum während 24 Stunden getrocknet. Der Rückstand wird mit Eisessig (30 ml) und Schwefelsäure (0,2 ml) versetzt und unter Argonatmosphäre während 30 Minuten bis zu einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um **3',4'-Dimethoxy-5-hydroxy-7-(3,4-dimethoxybenzoyl)oxyflavon (Luteolin Derivat)** zu ergeben; ¹H NMR (250MHz,CDCl₃): δ = 3,9 (s,12 H), 6,65-6,70 (m, 2 H), 6,95-7,00 (m, 3 H), 7,35 and 7,67 (AB dd, 1 H), 7,53 and 7,85 (AB dd, 2 H), 12,85 (s, 1 H); ¹³C NMR DEPT (250MHz, DMSO -d⁶): δ = 56,16 (s), 77,23 (s), 101,23 (s),104,96 (s), 105,61 (s), 108,83 (s), 110,51(s), 111,26 (s), 112,44 (s), 120,35 (s), 124,77 (s); EI-MS (70eV) *m*/*z* (rel. abund.) = 478; UV (2-propanol): λ = 271 nm, 344 nm.

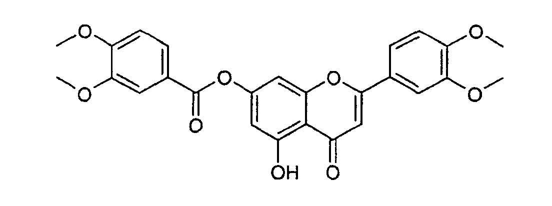

### Beispiel 2

Trockenes, pulverförmiges Lithiumhydroxid (18 mmol, 3,6 Äquivalente) wird auf einmal zu einer gut gerührten Lösung von 2,4,-Dihydroxyacetophenon (5 mmol) in trockenem THF (5 ml) unter Argonatmosphäre bei -78°C zugegeben. Die Reaktionsmischung wird bei - 78°C während einer Stunde gerührt und anschließend während zwei Stunden bei ca. 0°C. Nach erneutem Abkühlen auf -78°C, wird auf einmal eine Lösung von 3,4-Dimethoxybenzoesäurechlorid (5,9 mmol) in THF (10 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (150g) und konzentrierter HCl (5ml) geleert und mit Chloroform (3x50 ml) extrahiert. Die Lösungsmittel werden aus den getrockneten Extrakten entfernt und der Rückstand unter Vakuum während 24 Stunden getrocknet. Der Rückstand wird mit Eisessig (30 ml) und Schwefelsäure (0,2 ml) versetzt und unter Argonatmosphäre während 30 Minuten bis zu einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um **3',4'-Dimethoxy-7-hydroxyflavon** zu ergeben. ¹H NMR (250MHz, DMSO -d⁶): δ=3,85 (d, 6 H), 6,85-8,00 (m, 7 H), 10,72 (s, 1 H); ¹³C NMR (250MHz, DMSO -d⁶): δ= 56,08 (s), 56,22 (s), 102,95 (s), 105,82 (s), 109,76 (s), 112,11 (s), 115,19 (s), 116,51 (s), 119,38(s), 123,97 (s), 126,80 (s), 149,40 (s), 152,10 (s), 157,79 (s), 162,41 (s), 162,94 (s), 176,70 (s); EI-MS (70eV) *m*/*z* (rel. abund.) = 298; UV (2-propanol): λ = 236 nm, 332 nm.

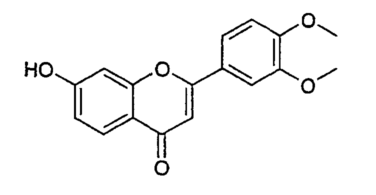

### Beispiel 3

Trockenes, pulverförmiges Lithiumhydroxid (19,7 mmol, 3 Äquivalente) wird auf einmal zu einer gut gerührten Lösung von 2,6-Dihydroxyacetophenon (6,6 mmol) in trockenem THF (5 ml) unter Argonatmosphäre bei -78°C zugegeben. Die Reaktionsmischung wird bei -78°C während einer Stunde gerührt und anschließend während zwei Stunden bei -10°C. Nach erneutem Abkühlen auf -78°C, wird auf einmal eine Lösung von 3,4-Dimethoxybenzoesäurechlorid (6 mmol) in THF (10 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (150g) und konzentrierter HCl (5ml) geleert und mit Chloroform (3x50 ml) extrahiert. Die Lösungsmittel werden aus den getrockneten Extrakten entfernt und der Rückstand unter Vakuum während 24 Stunden getrocknet. Der Rückstand wird mit Eisessig (30 ml) und Schwefelsäure (0,2 ml) versetzt und unter Argonatmosphäre während 30 Minuten bis zu einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um **3',4'-Dimethoxy-5-hydroxyflavone** zu ergeben. ¹H NMR (250MHz,CDCl₃): δ = 4,00 (d, 6 H), 6,60-7,60 (md, 7 H), 12,65 (s, 1 H);¹³C NMR CDP (250MHz, CDCl₃): δ = 54,71 (s), 103,48 (s), 105,57 (s), 107,45 (s), 109,76 (s), 118,93 (s), 122,24 (s), 133,80 (s), 147,93 (s), 151,06 (s), 159,39 (s), 163,17 (s), 182,04 (s);EI-MS (70eV) *m*/*z* (rel. abund.) = 298; UV (2-propanol): λ = 248 nm, 342,5 nm.

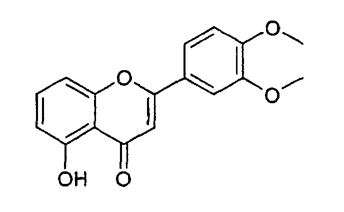

### Beispiel 4:

Trockenes, pulverförmiges Lithiumhydroxid (19,5 mmol, 3 Äquivalente) wird auf einmal zu einer gut gerührten Lösung von 2,6-Dihydroxyacetophenon (6,6 mmol) in trockenem THF (5 ml) unter Argonatmosphäre bei -78°C zugegeben. Die Reaktionsmischung wird bei -78°C während einer Stunde gerührt und anschließend während zwei Stunden bei -10°C. Nach erneutem Abkühlen auf -78°C, wird auf einmal eine Lösung von lsophthalatchlorid (3,3 mmol) in THF (10 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (150g) und konzentrierter HCl (5ml) geleert und mit Chloroform (3x50 ml) extrahiert. Die Lösungsmittel werden aus den getrockneten Extrakten entfernt und der Rückstand unter Vakuum während 24 Stunden getrocknet. Der Rückstand wird mit Eisessig (30 ml) und Schwefelsäure (0,2 ml) versetzt und unter Argonatmosphäre während 30 Minuten bis zu einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um **3'-(5-Hydroxychromon)yl-5-hydroxyflavon** zu ergeben. EI-MS (70eV) *m*/*z* (rel. abund.) = 398.

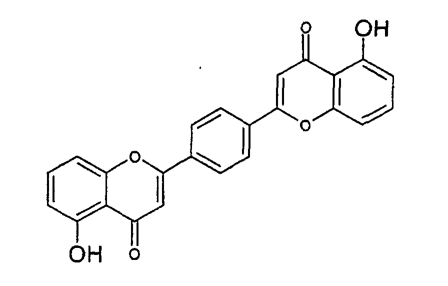

### Beispiel 5

Trockenes, pulverförmiges Lithiumhydroxid (19,7 mmol, 3 Äquivalente) wird auf einmal zu einer gut gerührten Lösung von 2',5'-Dihydroxyacetophenon (6,4 mmol) in trockenem THF (5 ml) unter Argonatmosphäre bei -78°C zugegeben. Die Reaktionsmischung wird bei -78°C während einer Stunde gerührt und anschließend während zwei Stunden bei -10°C. Nach erneutem Abkühlen auf -78°C, wird auf einmal eine Lösung von 4-Methoxybenzoesäurechlorid (6,5 mmol) in THF (10 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (150g) und konzentriert HCl (5ml) geleert und mit Chloroform (3x50ml) extrahiert. Die Lösungsmittel werden aus den getrockneten Extrakten entfernt und der Rückstand unter Vakuum während 24 Stunden getrocknet. Der Rückstand wird mit Eisessig (30 ml) und Schwefelsäure (0,2 ml) versetzt und unter Argonatmosphäre während 30 Minuten bis zur einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um 4'-Methoxy-6-hydroxyflavon zu ergeben. ¹H NMR (250 MHz, d⁶-DMSO): δ = 3,73 (s, 3H), 6,6-8,5 (m, 8H), 12,5 (s, 1OH); NMR (250 MHz, d⁶-DMSO): δ = 56 (s), 94,9 (s), 114,0 (s), 117,6 (s), 118,9 (s), 122,2 (s), 126,6(s), 127,2 (s), 150,5 (s), 152,0 (s), 161,2 (s), 168,8 (s), 187,0 (s) EI-MS (70eV) m/Z (rel. abund.)= 268; UV (2-propanol): λ = 254 nm, 323 nm.

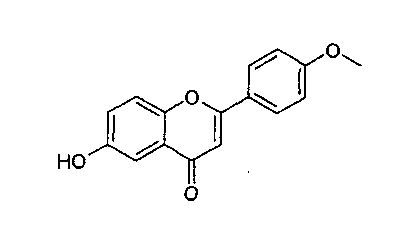

### Beispiel 6:

Trockenes, pulverförmiges Lithiumhydroxid (11,55 mmol, 3,3 Äquivalente) wird auf einmal zu einer gut gerührten Lösung von 2,4-Dihydroxyacetophenon (3,22 mmol) in trockenem THF (5 ml) unter Argonatmosphäre bei -78°C zugegeben. Die Reaktionsmischung wird bei -78°C während einer Stunde gerührt und anschließend während zwei bei -10°C. Nach erneutem Abkühlen auf -78°C, wird auf einmal eine Lösung von 9-Fluoreonon-4-carbonylchforid (3,3 mmol) in THF (10 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (150g) und konzentrierter HCl (5ml) geleert und mit Chloroform (3x50 ml) extrahiert. Die Lösungsmittel werden aus den getrockneten Extrakten entfernt und der Rückstand unter Vakuum während 24 Stunden getrocknet. Der Rückstand wird mit Eisessig (30 ml) und Schwefelsäure (0,2 ml) versetzt und unter Argonatmosphäre während 30 Minuten bis zu einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um **2-(9-Fluorenon-4-yl)-hydroxychromon** zu ergeben. EI-MS (70eV) *m*/*z* (rel. abund.) = 340.

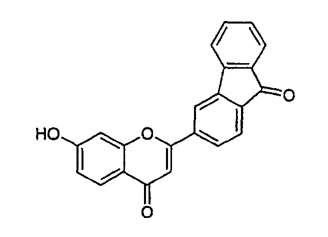

### Beispiel 7:

Trockenes, pulverförmiges Lithiumhydroxid (40 mmol, 2 Äquivalente) wird auf einmal zu einer gut gerührten Lösung von 2'-hydroxy-4',5'-dimethylenoxyacetophenon (20 mmol) in trockenem THF (100 ml) unter Argonatmosphäre bei -78°C zugegeben. Die Reaktionsmischung wird bei -78°C während einer Stunde gerührt und anschließend während zwei Stunden bei -10°C. nach erneutem Abkühlen auf -78°C, wird auf einmal eine Lösung von piperonylbenzoesäurechlorid (22 mmol) in THF (100 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (400 g) und konzentriert HCl (15 ml) geleert und mit Dichloromethan (4x60ml) extrahiert. Die Lösungsmittel werden aus den getrockneten Extrakten entfernt und der Rückstand unter Vakuum während 24 Stunden getrocknet. Der Rückstand wird mit Eisessig (220 ml) und Schwefelsäure (1,1 ml) versetzt und unter Argonatmosphäre während 30 Minuten bis zur einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um 3',4',6,7-Bis(dimethylenoxy)-flavon zu ergeben. EI-MS (70eV) m/Z (rel. abund.)= 310.

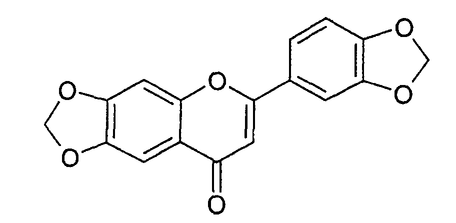

### Beispiel 8:

Trockenes, pulverförmiges Lithiumhydroxid (51 mmol, 4 Äquivalente) wird auf einmal zu einer gut gerührten Lösung von 2',6'-dihydroxyacetophenon (12,8 mmol) in trockenem THF (40 ml) unter Argonatmosphäre bei -78°C zugegeben. Die Reaktionsmischung wird bei -78°C während einer Stunde gerührt und anschließend während zwei Stunden bei -10°C. nach erneutem Abkühlen auf -78°C, wird auf einmal eine Lösung von 4-chlorbenzoesäurechlorid (13,97 mmol) in THF (20 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (400g) und konzentriert HCl (16 ml) geleert und mit dichloromethan (3x50ml) extrahiert. Die Lösungsmittel werden aus den getrockneten Extrakten entfernt und der Rückstand unter Vakuum während 24 Stunden getrocknet. Der Rückstand wird mit Eisessig (100 ml) und Schwefelsäure (0,5 ml) versetzt und unter Argonatmosphäre während 30 Minuten bis zur einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um 4'-chloro-5-hydroxyflavon zu ergeben. ¹H NMR (250 MHz, CDCl₃): δ = 6,7-8,0 (m, 7H), 12,5 (s, 10H); EI-MS (70eV) m/Z (rel. abund.)= 272; UV (2-propanol): λ = 213 nm, 275 nm, 301 nm, 338 nm.

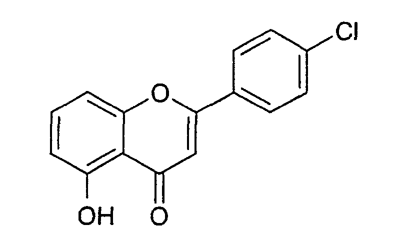

### Beispiel 9:

Trockenes, pulverförmiges Lithiumhydroxid (52,5 mmol, 4 Äquivalente) wird auf einmal zu einer gut gerührten Lösung von 2',6'-dihydroxyacetophenon (13,1 mmol) in trockenem THF (40 ml) unter Argonatmosphäre bei -78°C zugegeben. Die Reaktionsmischung wird bei -78°C während einer Stunde gerührt und anschließend während zwei Stunden bei -10°C. nach erneutem Abkühlen auf -78°C, wird auf einmal eine Lösung von 4-Nitrobenzoesäurechlorid (14,46 mmol) in THF (40 ml) zugegeben. Während einer Stunde wird bei -78°C und bei Raumtemperatur während 4 Stunden, bis zum Verschwinden des Ausgangsmaterials gerührt. Die Reaktionsmischung wird auf eine Mischung aus Eis (400 g) und konzentriert HCl (16 ml) geleert und mit dichloromethan (3x50ml) extrahiert. Die Lösungsmittel werden aus den getrockneten Extrakten entfernt und der Rückstand unter Vakuum während 24 Stunden getrocknet. Der Rückstand wird mit Eisessig (100 ml) und Schwefelsäure (0,5 ml) versetzt und unter Argonatmosphäre während 30 Minuten bis zur einer Stunde auf 95-100°C erhitzt. Etwa ein Drittel der Essigsäure wird abgezogen und der Rückstand in Wasser geleert. Das ausgefällte Produkt wird filtriert, gewaschen und getrocknet und in Methanol umkristallisiert um 4'-nitro-5-hydroxyflavon zu ergeben. ¹H NMR (250 MHz, CDCl₃): δ = 6,75-8,5 (m, 7H), 12,3 (s, 1OH); EI-MS (70eV) m/Z (rel. abund.)= 283.

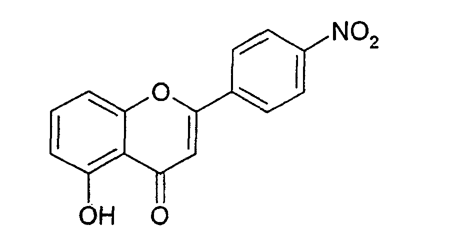

## Patentansprüche

1. Verfahren zur Herstellung von Flavon Derivaten, wobei man eine 2-Hydroxyacetophenon-Verbindung mit einer Lithiumverbindung metalliert und anschließend mit einer Ketoverbindung umsetzt, **dadurch gekennzeichnet, daß** das Verhältnis der molaren Äquivalente von Lithiumverbindung zu den zu metallierenden funktionellen Gruppen der 2-Hydroxyacetophenon-Verbindung 1 bis 1,2 beträgt und die Lithiumverbindung eine anorganische Lithiumverbindung ist.

2. Verfahren nach Anspruch 1, **dadurch gekennzeichnet, daß** das Verhältnis der molaren Äquivalente von Lithiumverbindung zu den zu metallierenden funktionellen Gruppen der 2-Hydroxyacetophenon-Verbindung genau 1 beträgt.

3. Verfahren nach einem der Ansprüche 1 bis 2, **dadurch gekennzeichnet, daß** die Metallierung in einem etherischen Lösungsmittel durchgeführt wird.

4. Verfahren nach einem der vorhergehenden Ansprüche, **dadurch gekennzeichnet, daß** das 2-Hydroxyacetophenon folgende Struktur aufweist: 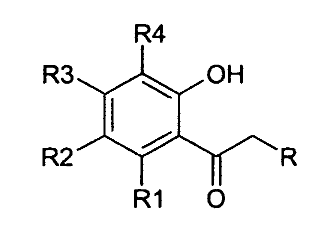 wobei R₁ bis R₄ Wasserstoff, eine funktionelle Gruppe, die aus Hydroxyl, Alkyl, Alkenyl, Ether, Ester, Aryl, O-glycosyl, Alkyloxy, Alkenoxy, Aryloxy, Halogen, Nitro oder Amino ausgewählt ist, bedeuten und gleich oder verschieden sein können und/oder für Rₙ-Rₙ₊₁ für n = 1, 2, 3, Teil eines Ringsystems sein können, und wobei R Wasserstoff, eine Alkoxy Gruppe, eine Phenyl- oder substituierte Phenylgruppe bedeutet.

5. Verfahren nach einem der vorhergehenden Ansprüche **dadurch gekennzeichnet, daß** die Ketoverbindung folgende Struktur aufweist: 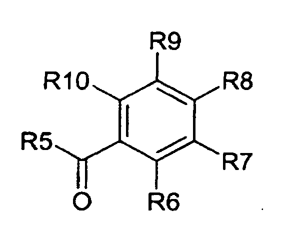 wobei R₆ bis R₁₀ Wasserstoff, eine funktionelle Gruppe, die aus Hydroxyl, Alkyl, Alkenyl, Ether, Ester, Aryl, O-glycosyl, Alkyloxy, Alkenoxy, Aryloxy, Halogen, Nitro oder Amino ausgewählt ist, bedeuten und gleich oder verschieden sein können und/oder wobei Rₙ-Rₙ₊₁ für n = 6, 7, 8, 9 Teil eines Ringsystems sein können, und wobei R₅ eine Halogenid-, Alkoxyl- oder Ester-Gruppe bedeuten kann.

6. Verfahren nach einem der vorhergehenden Ansprüche, **dadurch gekennzeichnet, daß** die Hydroxylgruppen der 2-Hydroxyacetophenon Verbindung ungeschützt sind.

## Claims

1. Process for the preparation of flavone derivatives in which a 2-hydroxyacetophenone compound is metallated using a lithium compound and subsequently reacted with a keto compound, **characterised in that** the ratio of the molar equivalents of lithium compound to the 2-hydroxyacetophenone compound functional groups to be metallated is 1 to 1.2, and the lithium compound is an inorganic lithium compound.

2. Process according to Claim 1, **characterised in that** the ratio of the molar equivalents of lithium compound to the 2-hydroxyacetophenone compound functional groups to be metallated is precisely 1.

3. Process according to one of Claims 1 to 2, **characterised in that** the metallation is carried out in an ethereal solvent.

4. Process according to one of the preceding claims, **characterised in that** the 2-hydroxyacetophenone has the following structure: 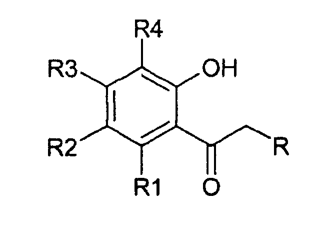 where R₁ to R₄ denote hydrogen, a functional group selected from hydroxyl, alkyl, alkenyl, ether, ester, aryl, O-glycosyl, alkoxy, alkenoxy, aryloxy, halogen, nitro or amino, and may be identical or different and/or, for Rₙ-Rₙ₊₁ for n = 1, 2, 3, may be part of a ring system, and where R denotes hydrogen, an alkoxy group, a phenyl or substituted phenyl group.

5. Process according to one of the preceding claims, **characterised in that** the keto compound has the following structure: 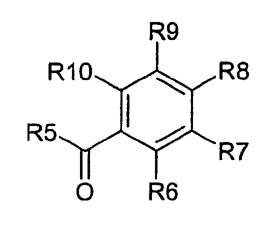 where R₆ to R₁₀ denote hydrogen, a functional group selected from hydroxyl, alkyl, alkenyl, ether, ester, aryl, O-glycosyl, alkoxy, alkenoxy, aryloxy, halogen, nitro and amino, and may be identical or different and/or where Rₙ-Rₙ₊₁, for n = 6, 7, 8, 9, may be part of a ring system, and where R₅ can denote a halide, alkoxy or ester group.

6. Process according to one of the preceding claims, **characterised in that** the hydroxyl groups of the 2-hydroxyacetophenone compound are unprotected.

## Revendications

1. Procédé pour la préparation de dérivés de flavone selon lequel un composé de 2-hydroxyacétophénone est métallisé en utilisant un composé de lithium et est ensuite amené à réagir avec un composé de céto, **caractérisé en ce que** le rapport des équivalents molaires de composé de lithium sur les groupes fonctionnels de composé de 2-hydroxyacétophénone à métalliser est de 1 à 1,2, et le composé de lithium est un composé de lithium inorganique.

2. Procédé selon la revendication 1, **caractérisé en ce que** le rapport des équivalents molaires de composé de lithium sur les groupes fonctionnels de composé de 2-hydroxyacétophénone à métalliser est précisément de 1.

3. Procédé selon l'une des revendications 1 à 2, **caractérisé en ce que** la métallisation est mise en oeuvre dans un solvant d'éther.

4. Procédé selon l'une des revendications précédentes, **caractérisé en ce que** le 2-hydroxyacétophénone présente la structure qui suit: 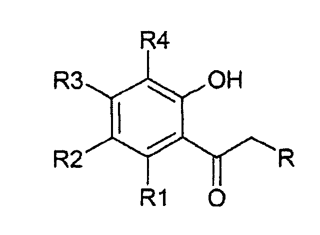 où R₁ à R₄ représentent hydrogène, un groupe fonctionnel choisi parmi hydroxyle, alkyle, alkényle, éther, ester, aryle, O-glycosyle, alkoxy, alkénoxy, aryloxy, halogène, nitro ou amino, et peuvent être identiques ou différents et/ou, pour Rₙ-Rₙ₊₁ pour n = 1, 2, 3, peuvent être une partie d'un système en anneau, et où R représente hydrogène, un groupe alkoxy, un groupe phényle ou phényle substitué.

5. Procédé selon l'une des revendications précédentes, **caractérisé en ce que** le composé de céto présente la structure qui suit:: 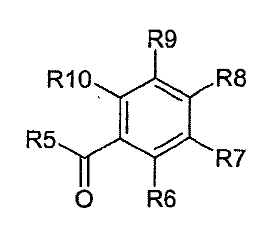 où R₆ à R₁₀ représentent hydrogène, un groupe fonctionnel choisi parmi hydroxyle, alkyle, alkényle, éther, ester, aryle, O-glycosyle, alkoxy, alkénoxy, aryloxy, halogène, nitro et amino, et peuvent être identiques ou différents et/ou où Rₙ-Rₙ₊₁, pour n = 6, 7, 8, 9, peuvent être une partie d'un système en anneau, et où R₅ peut représenter un groupe halogénure, alkoxy ou ester.

6. Procédé selon l'une des revendications précédentes, **caractérisé en ce que** les groupes hydroxyle du composé 2-hydroxyacétophénone sont non protégés.
